# EUROPÄISCHE PATENTSCHRIFT

(11) **EP 2 539 460 B1**
(45) Veröffentlichungstag und Bekanntmachung des Hinweises auf die Patenterteilung: **09.09.2015**
(21) Anmeldenummer: 11706794.2
(22) Anmeldetag: 21.02.2011
(51) Int. Cl.: C12Q 1/68

(54) **EINZELNUKLEOTID-POLYMORPHISMEN DES HUMANEN CHROMOSOMS 4 IM INOSITOL-POLYPHOSPHAT-4-PHOSPHATASE-TYP II-GEN (INPP4B-GEN) ZUR DIAGNOSE ODER PRÄDIAGNOSE VON MULTIPLER SKLEROSE**
MONONUCLEOTIDE POLYMORPHISMS OF THE HUMAN CHROMOSOME 4 IN THE INOSITOL POLYPHOSPHATE 4-PHOSPHATASE TYPE II GENE (INPP4B GENE) FOR DIAGNOSING OR PRE-DIAGNOSING MULTIPLE SCLEROSIS
POLYMORPHISMES D'UN SEUL NUCLÉOTIDE DU CHROMOSOME HUMAIN 4 DANS LE GÈNE INOSITOL-POLYPHOSPHATE-4-PHOSPHATASE TYPE II (GÈNE INPP4B) POUR LE DIAGNOSTIC OU LE PRÉDIAGNOSTIC DE LA SCLÉROSE EN PLAQUES

(30) Priorität: 22.02.2010 DE 102010008827
(43) Veröffentlichungstag der Anmeldung: 02.01.2013
(73) Patentinhaber: Immungenetics AG, 18055 Rostock (DE)
(72) Erfinder: IBRAHIM, Saleh, 23562 Lübeck (DE)
(74) Vertreter: Wablat Lange Karthaus
(86) Internationale Anmeldenummer: PCT/EP2011/052500
(87) Internationale Veröffentlichungsnummer: WO 2011/101466

(56) Entgegenhaltungen:
- WO-A1-2007/049118
- DATABASE dbSNP [Online] 20. März 2004 (2004-03-20), "Submitted SNP(ss) Details: ss22039633", XP002642015, Database accession no. rs13102150
- DATABASE NCBI Probe [Online] 2. Dezember 2006 (2006-12-02), "Bead microarray element (bead) probe for Homo sapiens variation rs2059510", XP002642016, gefunden im NCBI Database accession no. Pr006179158.1
- DATABASE NCBI Probe [Online] NCBI Probe pr002563124; 23. Januar 2006 (2006-01-23), "Sequence-specific oligonucleotide (SSO) probe for Homo sapiens variation rs17717651", XP002642017, Database accession no. Pr002563124.1
- CHRISTINA GEWINNER ET AL: "Evidence that Inositol Polyphosphate 4-Phosphatase Type II Is a Tumor Suppressor that Inhibits PI3K Signaling", CANCER CELL, Bd. 16, Nr. 2, 4. August 2009 (2009-08-04) , Seiten 115-125, XP055000590, ISSN: 1535-6108, DOI: 10.1016/j.ccr.2009.06.006
- IBRAHIM SALEH M ET AL: "Genomics, proteomics, metabolomics: what is in a word for multiple sclerosis?", CURRENT OPINION IN NEUROLOGY, Bd. 18, Nr. 3, Juni 2005 (2005-06), Seiten 231-235, XP009149332, ISSN: 1350-7540
- YU XINHUA ET AL: "mtDNA nt13708A variant increases the risk of multiple sclerosis.", PLOS ONE, Bd. 3, Nr. 2, E1530, Februar 2008 (2008-02), Seiten 1-7, XP002641738, ISSN: 1932-6203
- OCKINGER JOHAN ET AL: "Definition of a 1.06-Mb region linked to neuroinflammation in humans, rats and mice.", GENETICS, Bd. 173, Nr. 3, Juli 2006 (2006-07), Seiten 1539-1545, XP002641739, ISSN: 0016-6731

## Beschreibung

Die Erfindung betrifft einen Einzelnukleotid-Polymorphismus (SNP) der Nukleobase an Basenposition 143470133 (rs13102150) des humanen Chromosoms 4 im Inositol-poly-phosphat-4-phosphatase-Typ II-Gen (INPP4b-Gen) zur *in vitro* Diagnose oder Prädiagnose von Multipler Sklerose oder zur *in vitro* Ermittlung des Risikos, an Multipler Sklerose zu erkranken.

Erkrankungen, welche die Degeneration von Nervengewebe verursachen, umfassen eine Vielzahl unterschiedlicher Störungen bei jungen und alten Menschen. Solche Erkrankungen führen zu einer Abnahme funktional aktiver Neuronen im Gehirn und/oder im Rückenmark. Das Absterben der Neuronen kann verschiedene Ursachen haben: Ablagerung von Proteinen oder Proteinfragmenten, metabolisches Versagen, Gifte, Entzündungen, Durchblutungsstörungen oder Kopfverletzungen. Alle diese Faktoren verursachen einen weit reichenden Verlust von Neuronen und von Gliazellen, beispielsweise bei einem Schlaganfall. In anderen Fällen verursachen sie einen Verlust spezifischer Gruppen von Neuronen und funktioneller Systeme, beispielsweise bei Parkinson (PD) oder bei der Amyotrophen Lateralsklerose (ALS).

Allerdings sind die klinischen Merkmale bzw. Ausprägungen der einzelnen Krankheiten oftmals kaum zu unterscheiden. Dies führt zu Schwierigkeiten beim Versuch einer eindeutigen Diagnose. Zusätzlich variiert auch der Verlauf verschiedener Erkrankungen in großem Maße. Daher ist es bei einigen seltenen Erkrankungen oftmals erst im Rahmen einer Autopsie möglich, die genaue Krankheit zu bestimmen.

In der jungen Bevölkerung ist von den neurodegenerativen Erkrankungen die Multiple Sklerose (MS) am häufigsten vertreten. Die Multiple Sklerose ist eine chronisch-entzündliche Autoimunerkrankung des zentralen Nervensystems (ZNS). Die Krankheit zeichnet sich durch die Zerstörung der Myelinscheiden aus, welche die Axone der Nervenzellen umgeben. In frühe Stadien der MS erholen sich die Patienten oftmals vollständig von den Symptomen. Die strukturellen Schäden verbleiben jedoch und mit jedem weiteren Rezidiv erhöht sich die Wahrscheinlichkeit klinischer Schäden. Meistens beschränkt sich die Schädigung auf fokale Bereiche der weißen Substanz. Die strukturellen Veränderungen verursachen verschiedene klinische Symptome, beispielsweise visuelle Störungen, Gangprobleme, Ataxie, Parästhesie, Muskelschwäche und Parese, sowie Sprach- und Koordinationsprobleme, aber auch psychiatrische Schwierigkeiten. Die Ursache der MS ist immer noch unbekannt.

Die Krankheit beginnt oftmals bereits bei jungen Erwachsenen im Alter zwischen 20 und 30 Jahren, wobei deutlich mehr Frauen betroffen sind. MS ist eine Autoimmunerkrankung, welche sich strukturell durch Infiltration peripherer inflammatorischer Zellen auszeichnet, wobei es sich meistens um T- und B-Lymphozyten handelt. Diese Zellen finden sich in der weißen Substanz an lokalen Zerstörungspunkten, wo sie die Myelinscheiden angreifen. Aufgrund der Zerstörung des Myelins leidet die Isolierung der Axone. Im gesunden Zustand werden Aktionspotentiale durch die saltatorische Leitung an den Ranvier-Knoten geleitet. Diese Knoten weisen gar keine oder nur geringe Myelinierung zwischen zwei Oligodendrocyten an einem Axon auf. Eine beschädigte Isolierung stört nicht nur die saltatorische Leitung sondern führt aufgrund der Veränderungen in der lonenkonzentration auch zu metabolischen Problemen.

Zur Krankheitsursache von MS existieren lediglich einige Vermutungen, dass die genetische Prädisposition oder Umwelteinflüsse eventuelle eine Rolle beim Verlauf der Krankheit spielen könnten. Eine Beteiligung biologischer Faktoren wird ebenfalls diskutiert.

Insbesondere ein genetischer Indikator für MS wäre wünschenswert, da auf diesem Weg eine klare Diagnose möglich wäre. Bisherige Ansätze zeigen, dass ein erhöhtes Risiko für eine MS-Erkrankung mit dem Vorhandensein des humanen Leukozyenantigens (HLA) DR2 assoziiert ist. HLA-DR2 ist einer der definitiven genetischen Indikatoren für MS in der HLA-Region. Neben dem HLA-DR2-Haplotypus modulieren noch weitere Loki die Empfänglichkeit für MS in der HLA-Region, beispielsweise HLA-DR3. Genomische Studien weisen allerdings darauf hin, dass weitere genetische Faktoren zu einer Empfänglichkeit für MS beitragen. Es wird daher angestrebt, weitere genetische Indikatoren zu finden.

Aufgabe der vorliegenden Erfindung war es, weitere genetische Indikatoren zu finden, welche mit Multipler Sklerose assoziiert sind.

Im humanen Bereich wurde diese Aufgabe durch einen Einzelnukleotid-Polymorphismus (SNP) der Nukleobase an Basenposition 143470133 (rs13102150) des humanen Chromosoms 4 im Inositol-polyphosphat-4-phosphatase-Typ II-Gen (INPP4b-Gen) gelöst, welcher zur *in vitro* Diagnose oder Prädiagnose von Multipler Sklerose oder zur *in vitro* Ermittlung des Risikos, an Multipler Sklerose zu erkranken, dient bzw. hierfür Verwendung findet.

Die Suche nach Genen, welche bei einer polygenen Erkrankung wie MS beteiligt sind, ist sehr kompliziert und umfangreich. Eingesetzt wurde eine Kombination verschiedener Mapping-Strategien, wobei zuerst ein Tiermodell zum Einsatz kam. Die experimentelle autoimmune Encephalomyelitis (EAE) ist ein Tiermodell für MS. Dabei kann die Krankheit bei verschiedenen Spezies von der Maus bis hin zu höheren Primaten induziert werden. Das Tiermodell weist große Übereinstimmung mit der humanen Krankheit auf, beispielsweise akute, chronische Fehlfunktionen mit Rückfällen und Erholungsphasen, sowie Demyelinierung in der weißen Substanz des ZNS. Bei der Maus kann EAE auf zwei verschiedenen Wegen induziert werden, d.h. mittels aktiver oder adaptiver Immunisierung. Die aktive Impfung beinhaltet u.a. die Immunisierung mit Autoantigenen von Myelinproteinen oder mit Rückenmarkshomogenisaten. Die adaptive Immunisierung betrifft die Übertragung von Lymphknotenzellen von vorher immunisierten Mäusen oder von stimulierten antigen-spezifischen T-Zell-Linien.

Zur Analyse werden evozierte Potentiale (EPs) genutzt. Ein evoziertes Potential ist ein elektrisches Potential, welches bei einem Patienten nach einem Stimulus bis zur Erreichung des Erfolgsorgans aufgezeichnet wird. Beispielhaft zu nennen ist hier eine Muskelkontraktion. EPs zeigen, ob Läsionen vorhanden sind. Zur Diagnose können verschiedene EPs genutzt werden, beispielsweise visuell evozierte Potentiale (VEPs) oder mototisch evozierte Potentiale (MEPs). Zur Diagnose wurde hier die elektrische Stimulation des Gehirns zur Auslösung von motorisch evozierter Potentiale (MEP) in den Muskeln der Extremitäten genutzt. Aufgrund der Schädigung der Myelinscheide kommt es zu einer temporalen Dispersion der neuronalen Leitung, woraus veränderte corticale evozierte Antworten resultieren. Cortical motorisch evozierte Potentiale (cMEP) liefern quantitative Daten zum physiologischen Status und sind daher besonders geeignet für eine funktionale Untersuchung.

MS und EAE sind komplexe Erkrankungen, an denen viele Genorte (gen loci) beteiligt sind, die Auswirkung auf den Phänotyp haben können (quantitative trait loci, QTL). Ein QTL ist eine quantitative Merkmalstelle, d.h. ein variabler Punkt auf dem Genom, der ein bestimmtes Merkmal beeinflusst. Das Auffinden solcher Punkte ist sehr hilfreich bei der Suche nach Genen im Hinblick auf Krankheiten, an denen mehr als ein Gen beteiligt ist. Da das Merkmal nicht von einem einzelnen Gen ausgebildet wird, modifiziert jedes beteiligte Gen das Merkmal.

QTL-Mapping wird eingesetzt, um Kandidatengene für verschiedene Merkmale (traits) zu identifizieren. Bei der Maus werden für das QTL-Mapping Inzuchtstämme unter kontrollierten Umweltbedingungen verwendet, beispielsweise C57BI/6, SJL, FVB und C57BL/10.S.

Für das Mapping genetischer Loci werden verschiedene Marker verwendet, beispielsweise Fragment-Längen-Polymorphismen (RFLPs), hyervariable RFLPs, Minisatelliten, Microsatelliten oder Einzelnukleotidpolymorphismen (SNPs). Als relevant stellte sich der QTL EAE 31 heraus.

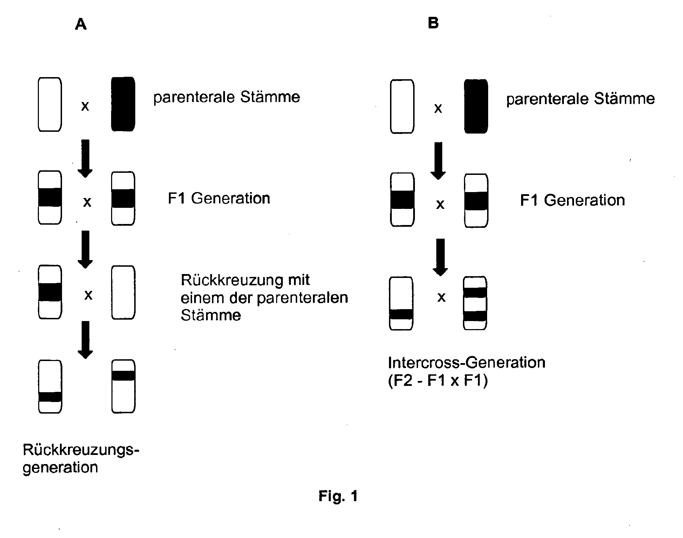

Da ein einzelner QTL hunderte von Genen enthalten kann, war die Auswahl einzelner Kandidatengene für die weitere Untersuchung anspruchsvoll. Daher war ein ergänzendes Fein-Mapping (fine mapping) unumgänglich, um den präzisen chromosomalen Locus zu identifizieren. Genutzt werden hierfür die Etablierung kongenetischer Linien (congenic lines), rekombinate Selektion und fortgeschrittene Intercross Linien (intercross lines). Die erste Option zur Erzeugung von Stämmen ist die Generierung von rückgekreuzten Populationen (backcross populations). Der zurückgekreuzte Stamm impliziert die Paarung von F1 Individuen mit einem der elterlichen Stämme (vgl. Fig. 1 B). Intercross-Generationen werden durch Paarung von Geschwistern der F1-Generation erzeugt, was eine gemischte F2-Population ergibt (vgl. Fig. 1 B). Die Analyse einer gemischten Population zweier Stämme auf verschiedene Phänotypen ist möglich. Für das Mapping des QTL und des chromosomalen Locus werden Softwarewerkzeuge angewandt.

Die Unterschiede zwischen verschiedenen Mäusestämmen können mittels hochdichter Marker oder durch die Schaffung von Stammverteilungsmustern analysiert werden. Daneben können Einzelnukleotidpolymorphismen (SNPs) auf phylogenetische Beziehungen zwischen Inzuchtstämmen hinweisen. Die Bestimmung, welches Fragment gleiche oder verschiedene Ahnen hat, wird durch Vergleich von SNPs verschiedener Mäusestämme in der QTL-Region möglich. Anhand der SNPs können Stämme in Haplotypen-Subgruppen unterteilt werden. Die SNPs helfen, diese Informationen mit den phänotypischen Daten zu kombinieren.

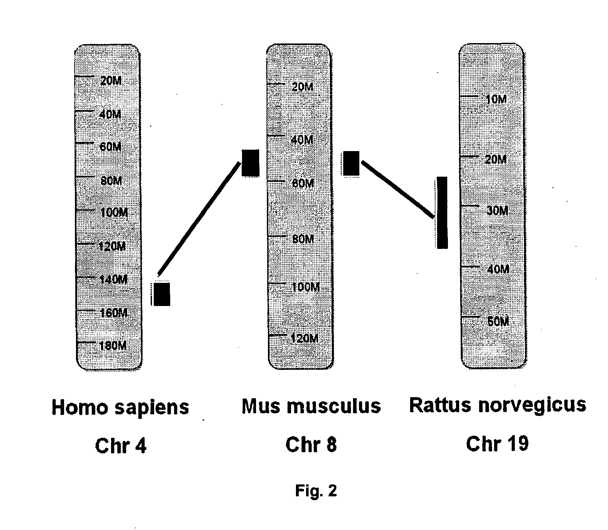

Ein anderer Ansatz ist die vergleichende Genomik (genomic). Anhand des genomischen Vergleichs verschiedener Spezies (beispielsweise Ratte, Maus, Mensch, vgl. Fig. 2) können gemeinsame Sequenzfragmente ermittelt werden. Weiterhin können der Genort, hochkonservierte Regionen oder die Menge an nicht-kodierender DNA ermittelt werden. Hochkonservierte Regionen innerhalb von Erkrankungsloci bei verschiedenen Spezien können mittels vergleichender Genomik bestimmt werden. Dies beschleunigt die Isolierung wahrscheinlicher Erkrankungsgene.

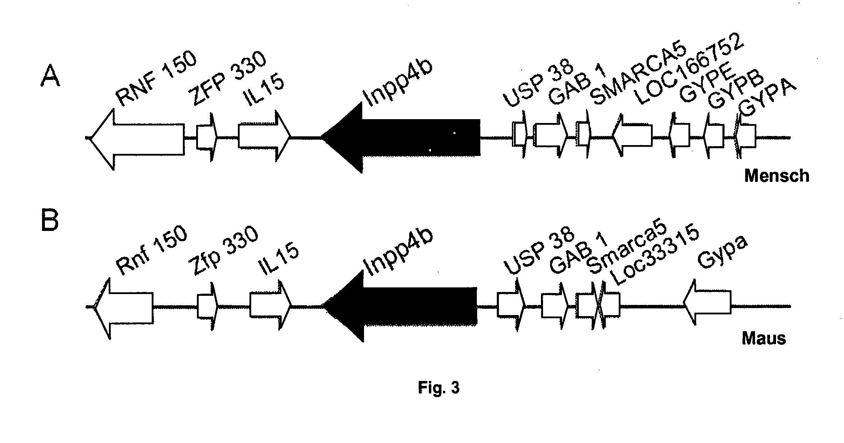

Für das Fein-Mapping des QTL (EAE 31) wurden die Haplotypen-Analyse, die intergenomische Analyse und das Genexpressionsprofilierung eingesetzt. Dabei wurde das Inositol-polyphosphat-4-phosphatase-Typ II-Gen (INPP4b-Gen) als das beste Kandidatengen auf Chromosom 8 der Maus ermittelt (vgl. Fig. 3).

Das Inpp4b (Protein) ist eine Mg²⁺-unabhängige Phosphatase, welche die Hydrolyse des Phosphats in 4-Postion des Phsophatidylinositol-3,4-biphosphats, des Inositol-1,3,4-triphosphats und des lnositol-3,4-biphosphats katalysiert. Das murine Protein ist zu 96% mit dem Menschen- und zu 90% zu dem Raten-Orthologen identisch.

Das Inpp4b-Gen als bestes Kandidatengen wurde in zwei Mausstämmen sequenziert, dem resistenten Stamm C57BL/10.S und dem empfänglichen Stamm SJL, um Unterschiede in der Sequenz zu finden. Der resistente Stamm widersteht einer EAE-Induzierung, der empfängliche Stamm reagiert auf die Induzierung mit EAE.

Die Sequenzunterschiede wurden mit bekannten Polymorphismen verglichen, welche zu Aminosäurevarianten beim Menschen führen. Die kodierende Sequenz des Inpp4b von SJL und von C57BL/10.S wurden in einem pGEM-T-easy-Vektor kloniert (Promega) und ergaben zwei SNP-Unterschiede in der cDNA, die zu einer Verschiebung von Aminosäuren (AS) führen: c1434C/A (AS 478 S/R) und c1655A/C (AS 552 H/P).

Um Herauszufinden, ob einer oder beide SNPs über die EAE-Anfälligkeit entscheiden, wurden DNA-Konstrukte erzeugt, welche jeweils eine Mutation (entweder Serin->Arginin oder Histidin->Prolin) oder beide Mutationen enthielten. Transgene Mäuse wurden per Pronuclei-Injektion erzeugt. Bei der Induzierung der EAE stellte sich heraus, dass beide SNPs relevant sind.

Das Inpp4b-Gen, welches bei der Maus auf Chromosom 8 lokalisiert ist, befindet beim Menschen auf Chromosom 4. Das Gen selbst ist bekannt, die Sequenz wird beispielsweise in Anderson et al., "The cDNA cloning and Characterization of Inositol Polyphosphatase 4-Phosphatase Typ II", J. Biolog. Chem. 1997, Vol. 272, Nr. 38, Seiten 23859-23864 wiedergegeben. Weiterhin ist das Gen beispielsweise in der ENSEMBL-Datenbank gelistet (Chromosom 4: 142,949,186-143,383,906). Es sind drei Splice-Varianten bekannt, d.h. alpha, alpha short und beta. Bei der alpha short-Variante fehlt das Exon 4.

Alle nachfolgenden Angaben zu den einzelnen SNPs beziehen sich auf die Definition bzw. Nummerierung gemäß Ensembl-Datenbank (Ensembl release 56 - Sept 2009; Homosapiensversion 56.37a (GRCh37)). Das Inpp4b-Gen bzw. die einzelnen Basen werden in Bezug auf die Orientierung des codogenen Strangs von Chromosom 4 abgelesen.

In einer Assoziationsstudie wurden MS-Patienten untersucht, wobei 39 SNPs als Marker in Frage kamen. DNA wurde aus Körperproben entnommen und im Bereich des Inpp4b-Gens wurde die jeweilige Sequenz identifiziert. An der Studie nahmen in der Kontrollgruppe insgesamt 349 Probanden teil, welche nicht an MS erkrankt sind, davon 210 Frauen und 152 Männer. In der Gruppe der erkrankten Patienten befanden sich 362 Personen, 4 davon wiesen ein klinisch isoliertes Syndrom auf, 8 waren primär progressiv, 3 progressiv rezidivierend, 244 waren in der Erholungsphase und 90 sekundär progressiv.

### Tag SNP Auswahl

Mittels des Tagger-Algorithmus' in Haploview wurden 39 Tag-SNPs ausgewählt, welche alle üblichen Haplotypen innerhalb des INPP4b-Gens abdecken (http://www/broad.-mit.edu/mpg/tagger, www.hapmap.org). Der Algorithmus basiert auf r². Die Anwendung eines stringenten r²-Grenzwertes (r² > 0,8) zwischen den SNPs erlaubt es, den ausgewählten Tag-SNPs, die meisten existierenden Haplotypen aufzulösen (siehe Altshuler D, Brooks LD, Chakravarti A, Collins FS, Daly MJ & Donnelly P 2005 International HapMap consortium a haplotype map of the human genome, Nature 437, 1299-1320; Barrett JC, Fry B, Maller J & Daly MJ 2005 Haploview: analysis and visualization of LD and haplotype maps, Bioinformatics 21, 263-265). Ausgewählt wurden SNPs mit minderen Allelhäufigkeiten (MAFs, minor allele frequencies) von mehr als 0,05.

### SNP Genotypisierung

Genomische DNA wurde aus peripheren Blutleukozyten unter Verwendung des QIAamp DNA Blood Mini Kits (Qiagen, USA) extrahiert. Die Genotypisierung aller SNPS erfolgte mittels eines 5'-Exonuclease-Assays (*Taq*Man assays on demand; Applied Biosystems, Inc., [ABI] Foster City, CA), wobei vom Hersteller zur Verfügung gestellte Primer verwendet wurden. Das Fluoreszenzsignal der Probe wurde gemäß Herstelleranweisung detektiert (*Taq*Man Assay for Real-Time PCR, 7500 Real Time PCR System; ABI).

Für jeden Probanden wurde der EDSS ermittelt, welcher ein Maß für die Schwere der Erkrankung darstellt (Expanded Disability Status Scale). Der Cochran-Armitage-Trend-Test wurde verwendet, um auf Assoziation zur Anfälligkeit für die Krankheit und zum EDSS-Wert zu testen. Die Ergebnisse für alle untersuchten SNPs sind in Tabelle 1 abgebildet.

Der Ausdruck "rsXXXXXXXX" steht für eine Bezeichnung gemäß Ensembl-Datenbank, das vom SNP betroffene Basenpaar auf dem humanen Chromosom 4 ist in der zweiten Spalte wiedergegeben, die P-Spalte gibt die erhaltenen Potentialwerte an. Die Spalte A1 gibt die normale Base an, die Spalte A2 die Base des SNPs. Signifikanz wiesen dabei rs13102150 [4:143470133 (codogener Strang, forward strand)], rs2059510 [4:143459907 (codogener Strang, forward strand)] und rs17717651 [4:143453079 (codogener Strang, forward strand)] auf, wobei insbesondere rs13102150 relevant war (Ensembl-Datenbankeinträge vom 11.01.2010).

**Tabelle 1**

| SNP | BP | A1 | A2 | P | OR | L95 | U95 |
|---|---|---|---|---|---|---|---|
| rs13102150 | 143470133 | C | A | 8.516E-03 | 0.729 | 0.576 | 0.923 |
| rs2874870 | 143509994 | C | T | 1.189E-02 | 0.722 | 0.56 | 0.932 |
| rs2636638 | 143230028 | A | G | 1.818E-02 | 0.741 | 0.577 | 0.951 |
| rs4975311 | 143500223 | G | A | 5.062E-02 | 0.777 | 0.602 | 1 |
| rs16998560 | 143481466 | G | C | 5.890E-02 | 0.783 | 0.607 | 1.01 |
| rs2667092 | 142963604 | C | G | 7.155E-02 | 0.746 | 0.542 | 1.03 |
| rs13107432 | 143444744 | A | G | 7.558E-02 | 0.811 | 0.644 | 1.02 |
| rs336307 | 143020338 | G | C | 8.171E-02 | 0.808 | 0.635 | 1.03 |
| rs1353624 | 143295246 | T | C | 1.310E-01 | 0.799 | 0.596 | 1.07 |
| rs2636637 | 143081071 | T | C | 1.488E-01 | 1.22 | 0.93 | 1.61 |
| rs1391098 | 143269745 | A | T | 1.875E-01 | 1.17 | 0.925 | 1.49 |
| rs2667101 | 142976133 | G | A | 1.881E-01 | 0.849 | 0.664 | 1.08 |
| rs2059510 | 143459907 | T | C | 1.998E-01 | 1.2 | 0.906 | 1.6 |
| rs2667096 | 142969773 | G | T | 2.138E-01 | 0.857 | 0.671 | 1.09 |
| rs336296 | 143014855 | T | C | 2.206E-01 | 0.849 | 0.654 | 1.1 |
| rs1219275 | 143084950 | T | C | 2.383E-01 | 1.17 | 0.901 | 1.52 |
| rs1391092 | 143341540 | T | A | 2.593E-01 | 0.869 | 0.681 | 1.11 |
| rs1995960 | 142916958 | A | G | 2.618E-01 | 0.877 | 0.698 | 1.1 |
| rs1907106 | 143229643 | C | T | 2.927E-01 | 1.13 | 0.898 | 1.43 |
| rs1497389 | 143304592 | C | A | 3.370E-01 | 1.13 | 0.882 | 1.44 |
| rs12499068 | 143343632 | C | T | 3.494E-01 | 0.894 | 0.706 | 1.13 |
| rs6821787 | 142920229 | G | T | 3.797E-01 | 0.879 | 0.659 | 1.17 |
| rs2635429 | 143243556 | C | T | 3.945E-01 | 1.11 | 0.876 | 1.4 |
| rs1391095 | 143294023 | A | T | 4.270E-01 | 1.11 | 0.861 | 1.43 |
| rs1817970 | 143364802 | G | A | 4.343E-01 | 0.872 | 0.619 | 1.23 |
| rs17717651 | 143453079 | C | A | 4.609E-01 | 1.12 | 0.833 | 1.5 |
| rs967003 | 142844783 | A | T | 5.069E-01 | 1.09 | 0.846 | 1.4 |
| rs2627798 | 143163452 | C | G | 5.102E-01 | 0.921 | 0.722 | 1.18 |
| rs1872292 | 143232882 | G | T | 5.507E-01 | 1.08 | 0.838 | 1.39 |
| rs168059 | 142999991 | T | G | 5.837E-01 | 0.932 | 0.725 | 1.2 |
| rs2636670 | 143234593 | A | G | 6.327E-01 | 1.06 | 0.837 | 1.34 |
| rs3756125 | 143344473 | T | C | 6.820E-01 | 1.05 | 0.824 | 1.34 |
| rs1994217 | 143130219 | T | A | 6.933E-01 | 0.953 | 0.748 | 1.21 |
| rs2029990 | 143277467 | C | T | 7.027E-01 | 1.05 | 0.82 | 1.34 |
| rs168061 | 143053786 | C | T | 7.221E-01 | 0.956 | 0.748 | 1.22 |
| rs1500847 | 142833334 | C | T | 7.994E-01 | 1.03 | 0.817 | 1.3 |
| rs3775707 | 143333792 | A | G | 8.607E-01 | 1.02 | 0.796 | 1.31 |
| rs2636645 | 143216769 | C | T | 8.664E-01 | 0.979 | 0.761 | 1.26 |
| rs3775692 | 143224971 | C | T | 9.781E-01 | 1 | 0.758 | 1.33 |

Entsprechend offenbart die Erfindung einen Einzelnukleotid-Polymorphismus (SNP) der Nukleobase an Basenposition 143470133 (rs13102150) des humanen Chromosoms 4 im Inositol-polyphosphat-4-phosphatase-Typ II-Gen (INPP4b-Gen) zur *in vitro* Diagnose oder Prädiagnose von Multipler Sklerose oder zur *in vitro* Ermittlung des Risikos, an Multipler Sklerose zu erkranken. An dieser Basenposition 143470133 findet sich normalerweise Cytosin. Diese Base ist im Falle eines SNPs durch eine andere Base -hier ein Adenin (A)- ersetzt. Unter einer "anderen Base" ist im gesamten Text zu verstehen, dass die Basen generell die Nukleobasen Adenin (A), Guanin (G), Cytosin (C) und Thymin (T) sind und dass der Ausdruck "andere Base" jeweils die Gruppe der verbleibenden drei Basen umfasst, d.h. beispielsweise wenn an an Basenposition 143470133 im Normalfall ein Cytosin vorliegt, können die anderen Basen Adenin, Guanin und Thymin sein, von denen eine dann anstelle des Cytosins vorliegt, wobei die Patentansprüche an der Basenposition 143470133 nur den Austausch von Cytosin gegen Adenin abdecken (rs13102150).

Die Erfindung betrifft somit einen Einzelnukleotid-Polymorphismus, welcher ein Austausch der Base Cytosin durch Adenin an Basenposition 143470133 (rs13102150) des humanen Chromosoms 4 im Inositol-polyphosphat-4-phosphatase-Typ II-Gen (INPP4b-Gen) ist, zur *in vitro* Diagnose oder Prädiagnose von Multipler Sklerose oder zur *in vitro* Ermittlung des Risikos, an Multipler Sklerose zu erkranken.

Für den Fall, dass an Basenposition 143470133 eine andere Base anstelle von Cytosin, hier Adenin anstelle von Cytosin, vorliegt, wird für den Patienten MS diagnostiziert bzw. prädiagnostiziert oder dem Patienten ein erhöhtes Krankheitsrisiko zugeordnet.

Die Testung hinsichtlich Assoziation mit EDSS erfolgte mittels Jonckheere-Terpstra-Test. Getestet wurden ausschließlich MS-Patienten. Wie bereits oben ausgeführt ist der "Expan-ded Disability Status Score" (EDSS) eine Leistungsskala, die Auskunft über den Schweregrad der Behinderung bei Multiple Sklerose-Patienten gibt. Die Skala beginnt bei 0 und endet bei 10, wobei die Schwere der Erkrankung mit ansteigendem Wert zunimmt. Der Arzt bezieht sich bei der Ermittlung der EDSS auf die Untersuchung der funktionellen Systeme (FS) des Patienten. Die Ergebnisse sind in Tabelle 2 abgebildet. Gezeigt sind die SNPs mit p < 0,1 aus dem MS-Assoziationstest oder dem Jonckhere-Terpstra-Test für Assoziation mit EDSS. Auch hier erwies sich insbesondere rs13102150 als relevant.

**Tabelle 2**

| SNP | pTrend | pJonckTerpstra |
|---|---|---|
| rs1391095 | 4.27E-01 | 3.60E-03 |
| rs2029990 | 7.03E-01 | 5.50E-03 |
| rs3775707 | 8.61E-01 | 6.70E-03 |
| rs3756125 | 6.82E-01 | 1.51E-02 |
| rs1872292 | 5.51E-01 | 1.63E-02 |
| rs1497389 | 3.37E-01 | 6.52E-02 |
| rs2635429 | 3.95E-01 | 6.92E-02 |
| rs2636670 | 6.33E-01 | 6.99E-02 |
| rs1391098 | 1.88E-01 | 8.21E-02 |
| rs2059510 | 2.00E-01 | 8.97E-02 |
| rs2874870 | 1.19E-02 | 9.64E-02 |
| rs2667092 | 7.16E-02 | 1.27E-01 |
| rs4975311 | 5.06E-02 | 2.23E-01 |
| rs13107432 | 7.56E-02 | 2.94E-01 |
| rs2636638 | 1.82E-02 | 3.25E-01 |
| rs13102150 | 8.52E-03 | 4.70E-01 |
| rs336307 | 8.17E-02 | 4.80E-01 |
| rs16998560 | 5.89E-02 | 7.69E-01 |

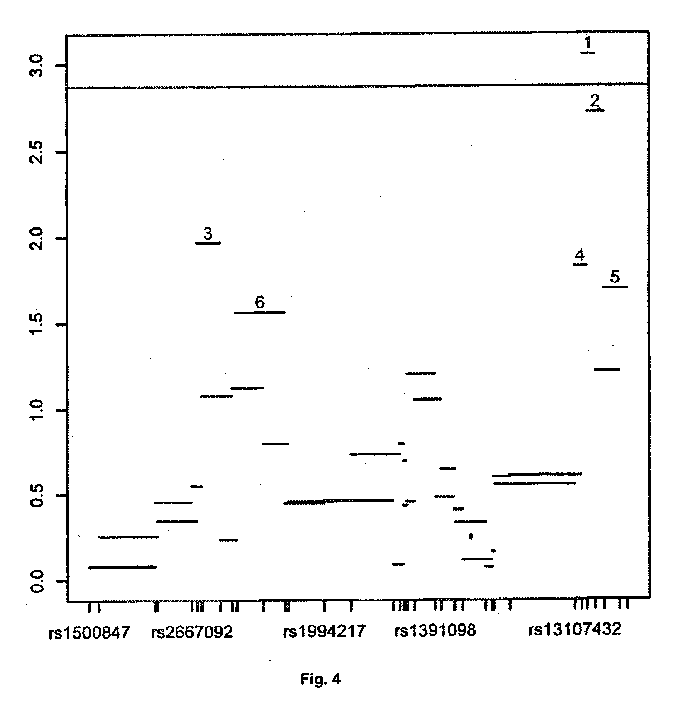

Die Haplotypus-Analyse für MS erfolgte mittels eines "sliding window approach", wobei die Window-Größe auf 3 gesetzt wurde. Das Ergebnis ist in Tabelle 3 und in Fig. 4 gezeigt. Von den Haplotypen 1 bis 6 erwies sich Haplotyp 1, welcher SNPs der Nukleobasen an Basenposition 143470133 (rs13102150), Basenposition 143459907 (rs2059510) und Basenposition 143453079 (rs17717651) des humanen Chromosoms 4 im Inositol-polyphosphat-4-phosphatase-Typ II-Gen (INPP4b-Gen) aufweist, als signifikant mit MS assoziiert.

**Tabelle 3**

| **Haplotyp** | pHaploScore | snp1 | snp2 | snp3 |
|---|---|---|---|---|
| 1 | 8.92E-04 | rs17717651 | rs2059510 | rs13102150 |
| 2 | 1.93E-03 | rs2059510 | rs13102150 | rs16998560 |
| 3 | 1.08E-02 | rs2667096 | rs2667101 | rs168059 |
| 4 | 1.49E-02 | rs13107432 | rs17717651 | rs2059510 |
| 5 | 2.02E-02 | rs16998560 | rs4975311 | rs2874870 |
| 6 | 2.74E-02 | rs336307 | rs168061 | rs2636637 |

Entsprechend ist eine bevorzugte Ausführungsform der Erfindung, dass ein Haplotyp, umfassend Einzelnukleotid-Polymorphismen (SNPs) der Nukleobasen an Basenposition 143470133 (rs13102150), Basenposition 143459907 (rs2059510) und Basenposition 143453079 (rs17717651) des humanen Chromosoms 4 im Inositol-polyphosphat-4-phosphatase-Typ II-Gen (INPP4b-Gen) zur *in vitro* Diagnose oder Prädiagnose von Multipler Sklerose oder zur *in vitro* Ermittlung des Risikos, an Multipler Sklerose zu erkranken, verwendet wird.

Dieser Haplotyp ist dadurch gekennzeichnet, dass die Polymorphismen einen Austausch der Base Cytosin durch Adenin an Basenposition 143470133 (rs13102150), einen Austausch der Base Thymin durch Cytosin an Basenposition 143459907 (rs2059510) und ein Austausch der Base Cytosin durch Adenin an Basenposition 143453079 (rs17717651) umfassen.

In anderen Worten wird bei einem Haplotyp beim dem an Basenposition 143470133 eine andere Base anstelle von Cytosin, hier Adenin anstelle von Cytosin, an Basenposition 143459907 eine andere Base anstelle von Thymin, hier Cytosin anstelle von Thymin, und an Basenposition eine andere Base anstelle von Cytosin, hier Adenin anstelle von Cytosin, vorliegt, für den Patienten MS diagnostiziert bzw. prädiagnostiziert oder dem Patienten ein erhöhtes Krankheitsrisiko zugeordnet.

Bei dem erfindungsgemäßen *in vitro* Verfahren zur Diagnose oder Prädiagnose von MS oder zur Ermittlung des Risikos eines Probanden, an MS zu erkranken, wird zumindest die Base an Basenposition 143470133 (rs13102150) des humanen Chromosoms 4 im Inositol-polyphosphat-4-phosphatase-Typ II-Gen (INPP4b-Gen) analysiert, wobei für den Fall, dass dort eine andere Base als Cytosin, hier ein Adenin anstelle eines Cytosins vorliegt, für den Probanden Multipler Sklerose diagnostiziert wird bzw. dem Probanden ein erhöhtes Erkrankungsrisiko zugeordnet wird.

In einer bevorzugten Ausführungsform des *in vitro* Verfahren zur Diagnose oder Prädiagnose von Multipler Sklerose oder zur Ermittlung des Risikos eines Probanden, an Multipler Sklerose zu erkranken, werden zumindest die Basen an Basenposition 143470133 (rs13102150), Basenposition 143459907 (rs2059510) und Basenposition 143453079 (rs17717651) des humanen Chromosoms 4 im Inositol-polyphosphat-4-phosphatase-Typ II-Gen (INPP4b-Gen) analysiert, wobei für den Fall, dass
an Basenposition 143470133 eine andere Base anstelle eines Cytosins, hier ein Adenin anstelle eines Cytosins
und
an Basenposition143459907 eine andere Base anstelle eines Thymins, hier ein Cytosin anstelle eines Thymins
und
an Basenposition 143453079 eine andere Base anstelle eines Cytosins, hier ein Adenin anstelle eines Cytosins, vorliegt,
für den Probanden Multiple Sklerose diagnostiziert wird bzw. dem Probanden ein erhöhtes Erkrankungsrisiko zugeordnet wird.

Bei dem erfindungsgemäßen *in vitro* Verfahren wird dem Probanden Körpermaterial, vorzugsweise Zell- und/oder Gewebematerial, entnommen. Besonders bevorzugt werden Blutproben entnommen. Daraus wird die DNA als Träger der genetischen Information isoliert und anschließend die Sequenz identifiziert, sowie mit der Referenzsequenz an der entsprechenden Stelle des humanen Chromosoms 4 bzw. des Inpp4b-Gens verglichen. Zur Identifizierung der Sequenz eignen sich vielfältige, den Fachmann bekannte Verfahren, welche auch eine Sequenzierung der DNA umfassen. Für Verfahren, welche eine DNA-Vervielfältigung erfordern, kann die Amplifikation zumindest eines Teils des Gens mit dem Fachmann bekannten Verfahren erfolgen. Beispielhaft zu nennen sind hier PCR und/oder LCR. Alternativ zu nennen sind Verfahren wie die "self sustained sequence replication", transkriptionelle Amplifikationssysteme oder Q-Beta Replikasen.

Da eine Sequenzierung sehr aufwendig ist, werden zur Identifikation bevorzugt Verfahren genutzt, welche keine vollständige Sequenzierung erfordern. Zu nennen sind hierfür Methoden wie Pyrosequencing-Verfahren, welche beispielsweise von der Firma QIAGEN angeboten werden, spezifische Verfahren zur Detektion von DNA-Unterschieden wie die Taqman®-PCR (Echtzeit-PCR-basierende Assays (Real-Time PCR-Based Assays), welche beispielsweise von der Firma AB applied biosystems angeboten werden, oder elektrochemische Ansätze zur DNA-Detektion, wie beispielsweise dem GENSORIC® der Firma Gensoric GmbH. Weitere Methoden zur Identifikation, welche angewandt werden, sind in EP 1 388 589 A1 beschrieben (Absätze [0111] ff.).

### Beschreibung der Figuren

- Fig. 1: Dargestellt sind zwei der angewandten Fein-Mapping-Strategien. In Teil A der Abbildung ist eine F1-Rückkreuzung mit einem parenteralen Stamm (FO) gezeigt. In Teil B der Abbildung ist das F1-Intercrossing mit einem Geschwister (F1) gezeigt.
- Fig. 2: Abgebildet ist der Vergleich chromosomaler Fragmente von Mensch, Maus und Ratte. Dargestellt ist die Lokation des QTL EAE 31 bei allen drei Spezien.
- Fig. 3: Abgebildet ist schematisch das EAE 31 QTL beim Menschen (A) und bei der Maus (B). Das Fein-Mapping des EAE 31 weist auf das Gen Inpp4b hin.
- Fig. 4: Gezeigt ist die Haplotypus-Analyse, angeben sind globale p-Werte für Sub-Haplotypen, basierend auf Tabelle 3. Die Linie zwischen 2.5 und 3.0 zeigt den Signifikanz-Grenzwert nach Korrektur für mehrfache Testung.

## Patentansprüche

1. *In vitro* Verfahren zur Diagnose oder Prädiagnose von Multipler Sklerose oder zur Ermittlung des Risikos eines Probanden, an Multipler Sklerose zu erkranken, **dadurch gekennzeichnet, dass** zumindest die Base an Basenposition 143470133 (rs13102150) des humanen Chromosoms 4 im Inositol-polyphosphat-4-phosphatase-Typ II-Gen analysiert wird, wobei für den Fall, dass dort ein Adenin anstelle eines Cytosins vorliegt, für den Probanden Multipler Sklerose diagnostiziert wird bzw. dem Probanden ein erhöhtes Erkrankungsrisiko zugeordnet wird.

2. *In vitro* Verfahren zur Diagnose oder Prädiagnose von Multipler Sklerose oder zur Ermittlung des Risikos eines Probanden, an Multipler Sklerose zu erkranken nach Anspruch 1, **dadurch gekennzeichnet, dass** für den Fall, dass
an Basenposition 143470133 (rs13102150) ein Adenin anstelle eines Cytosins und
an Basenposition143459907 (rs2059510) ein Cytosin anstelle eines Thymins und
an Basenposition 143453079 (rs17717651) ein Adenin anstelle eines Cytosins vorliegt,
für den Probanden Multiple Sklerose diagnostiziert wird bzw. dem Probanden ein erhöhtes Erkrankungsrisiko zugeordnet wird.

3. *In vitro* Verfahren nach einem der Ansprüche 1-2, **dadurch gekennzeichnet, dass** dem Probanden entnommenes Körpermaterial, vorzugsweise Zell- und/oder Gewebematerial, analysiert wird.

4. *In vitro* Verfahren nach einem der Ansprüche 1-3, **dadurch gekennzeichnet, dass** dem Probanden entnommene Blutproben analysiert werden.

5. *In vitro* Verfahren nach einem der Ansprüche 1 bis 4, **dadurch gekennzeichnet, dass** die zu untersuchende DNA aus vom Probanden entnommenen Körpermaterial, vorzugsweise Zell- und/oder Gewebematerial, vorzugsweise aus Blutproben, isoliert und anschließend die Sequenz identifiziert wird.

## Claims

1. An *in vitro* method for diagnosing or pre-diagnosing multiple sclerosis or for determining the risk of a proband developing multiple sclerosis, **characterized in that** at least the base at base position 143470133 (rs13102150) of human chromosome 4 in the inositol polyphosphate-4-phosphatase type II gene is analysed, wherein, if an adenine is present there instead of a cytosine, the proband is diagnosed with multiple sclerosis or the proband is categorized as being at increased risk of developing the disease.

2. The *in vitro* method for diagnosing or pre-diagnosing multiple sclerosis or for determining the risk of a proband developing multiple sclerosis according to claim 1, **characterized in that**, in the event that
an adenine instead of a cytosine is present at base position 143470133(rs13102150),
and
a cytosine instead of a thymine is present at base position 143459907 (rs2059510),
and
an adenine instead of a cytosine is present at base position 143453079 (rs177717651),
the proband is diagnosed with multiple sclerosis or the proband is categorized as being at increased risk of developing the disease.

3. The *in vitro method* according to one of claims 1-2, **characterized in that** bodily material taken from the proband, preferably cell and/or tissue material, is analysed.

4. The *in vitro method* according to one of claims 1-3, **characterized in that** blood samples taken from the proband are analysed.

5. The *in vitro method* according to one of claims 1 to 4, **characterized in that** the DNA to be analysed is isolated from the proband's bodily material, preferably cell and/or tissue material, preferably from blood samples, and the sequence is then identified.

## Revendications

1. Procédé *in vitro* de diagnostic ou pré-diagnostic de sclérose multiple ou de détermination du risque d'une personne testée de contracter une sclérose multiple, **caractérisé en ce qu'**au moins la base est analysée à la position de base 143470133 (rs13102150) du chromosome humain 4 dans le gène de type II d'inositol-polyphosphate-4-phosphatase, sachant que, dans le cas où on y est en présence d'une adénine à la place d'une cytosine, une sclérose multiple est diagnostiquée pour la personne testée ou un risque accru de contraction de la maladie est associé à la personne testée.

2. Procédé *in vitro* de diagnostic ou pré-diagnostic de sclérose multiple ou de détermination du risque d'une personne testée de contracter une sclérose multiple, selon la revendication 1, **caractérisé en ce que**,
dans le cas où on est en présence, à la position de base 143470133 (rs13102150), d'une adénine à la place d'une cytosine et,
à la position de base 143459907 (rs2059510), d'une cytosine à la place d'une thymine et,
à la position de base 143453079 (rs17717651), d'une adénine à la place d'une cytosine,
une sclérose multiple est diagnostiquée pour la personne testée ou un risque accru de contraction de la maladie est associé à la personne testée.

3. Procédé *in vitro* selon une des revendications 1 et 2, **caractérisé en ce que** le matériel physiologique prélevé sur la personne testée, de préférence le matériel cellulaire et/ou tissulaire, est analysé.

4. Procédé *in vitro* selon une des revendications 1 à 3, **caractérisé en ce que** des échantillons de sang prélevés sur la personne testée sont analysés.

5. Procédé *in vitro* selon une des revendications 1 à 4, **caractérisé en ce que** l'ADN à étudier dans le matériel physiologique prélevé sur la personne testée, de préférence le matériel cellulaire et/ou tissulaire, provenant de préférence de prélèvements sanguins, est isolé puis que la séquence est identifiée.
